# EUROPÄISCHE PATENTSCHRIFT

(11) **EP 1 807 842 B1**
(45) Veröffentlichungstag und Bekanntmachung des Hinweises auf die Patenterteilung: **11.12.2013**
(21) Anmeldenummer: 05806612.7
(22) Anmeldetag: 13.10.2005
(51) Int. Cl.: G21C 17/028, G01N 1/22, G01N 1/10

(54) **VERFAHREN UND PROBENAHMESYSTEM ZUR GEWINNUNG EINER PROBE AUS DER ATMOSPHÄRE IN EINEM REAKTORSICHERHEITSBEHÄLTER EINER KERNTECHNISCHEN ANLAGE**
METHOD AND SAMPLING SYSTEM FOR THE TAKING OF A SAMPLE FROM THE ATMOSPHERE IN A REACTOR CONTAINMENT HOUSING OF A NUCLEAR PLANT
PROCEDE ET SYSTEME D'ECHANTILLONNAGE POUR PRODUIRE UN ECHANTILLON DE L'ATMOSPHERE PRESENTE DANS UN RECIPIENT DE SURETE DU REACTEUR D'UNE INSTALLATION NUCLEAIRE

(30) Priorität: 14.10.2004 DE 102004050308
(43) Veröffentlichungstag der Anmeldung: 18.07.2007
(73) Patentinhaber: AREVA GmbH, 91052 Erlangen (DE)
(72) Erfinder: HILL, Axel, 64589 Stockstadt (DE); ECKARDT, Bernd, 63486 Bruchköbel (DE)
(74) Vertreter: Tergau & Walkenhorst
(86) Internationale Anmeldenummer: PCT/EP2005/011024
(87) Internationale Veröffentlichungsnummer: WO 2006/042691

(56) Entgegenhaltungen:
- EP-A- 0 129 110
- DE-A1- 2 309 659
- DE-A1- 4 126 894
- US-A- 5 367 546
- US-A- 6 074 882
- PATENT ABSTRACTS OF JAPAN Bd. 006, Nr. 062 (P-111), 21. April 1982 (1982-04-21) -& JP 57 004535 A (TOSHIBA CORP), 11. Januar 1982 (1982-01-11)
- DATABASE WPI Section EI, Week 197801 Derwent Publications Ltd., London, GB; Class S03, AN 1978-A1558A XP002364906 & SU 549 725 A (TATAR PETR EQUIP) 16. Mai 1977 (1977-05-16)
- PATENT ABSTRACTS OF JAPAN Bd. 1998, Nr. 12, 31. Oktober 1998 (1998-10-31) & JP 10 185116 A (ISHIKAWAJIMA HARIMA HEAVY IND CO LTD), 14. Juli 1998 (1998-07-14)

## Beschreibung

Die Erfindung betrifft eine kerntechnische Anlage mit einem Reaktorsicherheitsbehälter und mit einem zur Gewinnung einer Probe aus der Atmosphäre im Reaktorsicherheitsbehälter ausgelegten Probenahmesystem gemäß dem Oberbegriff des Anspruchs 1. Sie bezieht sich weiter auf ein entsprechendes Verfahren zur Gewinnung einer derartigen Probe.

In einer kerntechnischen Anlage könnte bei Stör- und insbesondere Unfallsituationen nach einem Störfall mit Kühlmittelverlust, signifikante Aktivitätsfreisetzung auftreten. Dabei kann insbesondere Wasserstoffgas innerhalb des den Reaktorkern umschließenden Sicherheitsbehälters oder Containments gebildet und freigesetzt werden, wobei aufgrund der möglichen Bildung explosiver Gasgemische eine Gefährdung des Reaktorsicherheitsbehälters durch auftretende unkontrollierte Wasserstoffreaktionen entstehen könnte.

Zur Verhinderung der Bildung derartiger explosiver Gasgemische im Containment oder Reaktorsicherheitsbehälter einer kerntechnischen Anlage sind daher verschiedene Konzepte bekannt, bei denen bedarfsgerecht eine Inertisierung der Atmosphäre im Reaktorsicherheitsbehälter vorgenommen wird. Dabei kann beispielsweise die kontrollierte Zündung oder Verbrennung sich bildender Wasserstoffanteile in der Containment-Atmosphäre vorgesehen sein. Dabei wird der Wasserstoffanteil zuverlässig abgebaut, bevor er Im Gasgemisch die Zündgrenze übersteigt, oberhalb derer eine unkontrollierte Wasserstoffreaktion auftreten könnte. Alternativ oder zusätzlich kann auch die kontrollierte Einspeisung von Inertgasen, wie beispielsweise Stickstoff, in die Atmosphäre des Reaktorsicherheitsbehälters vorgesehen sein, so dass bereits aufgrund des hohen Inertgasanteils in der Containmentatmosphäre eine Zündung des Gasgemisches ausgeschlossen ist.

Für eine kontrollierte und bedarfsgerechte Behandlung derartiger Unfallsituationen, also beispielsweise für eine gezielte Zuführung von Inertisierungsgasen, ist aber eine vergleichsweise zuverlässige Ermittlung des jeweils aktuellen Ist-Zustands in der Containment-Atmosphäre erforderlich. Aufgrund der in den genannten Störfallsituationen zu erwartenden vergleichsweise aggressiven Bedingungen für Bauteile und Komponenten infolge eventueller Strahlenbelastung und/oder chemischer Reaktivität von Atmosphärenbestandteilen ist die Überwachung der Containment-Atmosphäre und ihrer Bestandteile anhand von direkt erfassten Ist-Messwerten durch Mess- oder Analysesysteme innerhalb des Reaktorsicherheitsbehälters nicht mit hinreichender Genauigkeit und Zuverlässigkeit möglich. Um dennoch als geeignete Grundlage für die Steuerung erforderlicher Gegenmaßnahmen den aktuellen Ist-Zustand der Containment-Atmosphäre geeignet berücksichtigen zu können, kann aber eine sogenannte Probenahme vorgesehen sein, bei der eine, auch als Probe bezeichnete, geringe Teilmenge der Containment-Atmosphäre aus dem Reaktorsicherheitsbehälter entnommen und einer außerhalb des Sicherheitsbehälters angeordneten Analyse- und Auswertstation zugeführt wird. Ein zur Gewinnung einer derartigen Probe geeignetes Verfahren und eine zur Durchführung des Verfahrens geeignete Vorrichtung sind beispielsweise aus der DE 41 26 894 A1 bekannt.

Bei derartigen, bekannten Probenahmesystemen wird üblicherweise in einem außerhalb des Reaktorsicherheitsbehälters angeordneten Analysenschrank eine Messgastrocknung vorgenommen, wobei anschließend mit einem Wärmeleitfähigkeitsanalysator die Wasserstoffkonzentration des getrockneten Gases gemessen wird. Um aus diesem Messwert die im Reaktorsicherheitsbehälter herrschende tatsächliche Wasserstoffkonzentration zu bestimmen, wird eine Korrektur mit dem Dampfgehalt innerhalb der Containment-Atmosphäre durchgeführt. Diese Korrektur wird üblicherweise unter der Annahme von Sättigungsbedingungen mit dem Containmentdruck und der Containment-temperatur vorgenommen. Der in der Containment-Atmosphäre vorliegende wahre Dampfgehalt und die tatsächliche Wasserstoffkonzentration können wegen möglicherweise vorliegender Überhitzungen dabei nur mit unzureichender Genauigkeit bestimmt werden. Die sehr unterschiedlichen Atmosphärenzustände von Sättigungsbedingungen bis zum Zustand "stark überhitzt" werden verursacht durch die in der Atmosphäre befindlichen Anteile aktiver Edelgase und aerosolförmiger Spaltprodukte, die je nach Unfallverlauf eine Nachzerfallsieistung von wenigen kW bis hin zu mehreren 100 kW ausmachen können. Weiterhin ist zu berücksichtigen, dass auch in den verschiedenen Raum- und Höhenbereichen des Sicherheitsbehälters, bedingt durch Kühleffekte der Strukturen, der Außenwände, Kühlsysteme etc. erhebliche Temperaturunterschiede verursacht werden können. Die tatsächlichen Atmosphärentemperaturen können deshalb z. B. 0 bis >100°C von den Sättigungstemperaturen abweichen und lassen deshalb keinen zuverlässigen Rückschluss auf die tatsächlichen Dampfpartialdrücke zu.

Alternativ können auch Wasserstoffsensoren in Reaktorsicherheitsbehälter direkt eingesetzt sein, die nach dem Wärmetönungsprinzip arbeiten. Diese Sensoren können mittels störfallfester - jedoch nicht unfallfester - Kabel mit einer außerhalb des Reaktorsicherheitsbehälters angeordneten Messelektronik verbunden sein. Eine Wasserstoffmessung ist dabei jedoch allein durch diese Messeinrichtung, bei reduzierten Sauerstoffanteilen und insbesondere bei mittel- und langfristig hohen Strahlenbelastungen nicht mehr möglich. Unter inerten Bedingungen ist somit keine zuverlässige Messung der Wasserstoffkonzentration mehr möglich, wobei zudem eine vergleichsweise große Querempfindlichkeit zu Kohlenmonoxid besteht, das bei einer Wechselwirkung von Beton mit Kernschmelze freigesetzt werden könnte. Gerade beim aktiven Störfallmanagement und der gezielten Steuerung von Gegenmaßnahmen sind derartige Systeme daher nur unzureichend.

Des Weiteren können in bekannten Probenahmesystemen üblicherweise nur Einzelgasanteile, wie beispielsweise der Wasserstoff- oder der Sauerstoffanteil, analysiert werden, wobei eine direkte Bestimmung des Inertisierungszustands der Containment-Atmosphäre durch direkte Messung des Dampf- oder Kohlendioxidgehalts nicht durchgeführt werden kann.

Der Erfindung liegt daher die Aufgabe zugrunde, eine kerntechnische Anlage der genannten Art mit einem Probenahmesystem zur Gewinnung einer Probe aus der Atmosphäre im Reaktorsicherheitsbehälter anzugeben, mit dem die Bereitstellung einer zur Ermittlung besonders zuverlässiger und genauer Messwerte für Gasanteile der Containment-Atmosphäre geeigneten Probe ermöglicht ist. Des Weiteren soll ein zur Gewinnung einer derartigen Probe besonders geeignetes Verfahren angegeben werden. Bezüglich der kerntechnischen Anlage wird diese Aufgabe erfindungsgemäß gelöst durch die Merkmale des Anspruchs 1.

Die Erfindung geht dabei von der Überlegung aus, dass zur Ermittlung von für den aktuellen Ist-Zustand der Containment-Atmosphäre charakteristischen, besonders genauen Messwerte die bereitgestellte Probe die Atmosphärenbedingungen innerhalb des Containments mit besonders hoher Genauigkeit wiedergeben sollte. Dazu sollten diejenigen Einflüsse, die eine Verfälschung der Probenzusammensetzung im Vergleich zur tatsächlichen Ist-Zusammensetzung der Atmosphäre im Containment bewirken könnten, konsequent vermieden werden. Wie sich herausgestellt hat, könnte als eine mögliche Quelle für eine derartige Abweichung der Eigenschaften der entnommenen Probe von den Eigenschaften der tatsächlichen Containment-Atmosphäre in Unfallsituationen die üblicherweise vorgenommene Trocknung des Messgases und die anschließende Berücksichtigung der Einflüsse des Dampfgehalts unter der Annahme von Sättigungsbedingungen im Containment angesehen werden. Bei wesentlichen Aktivitäts- und Wasserstofffreisetzungen und der Annahme von Sattdampfbedingungen auf Basis der gemessenen Atmosphärentemperaturen führt dies leicht zu einem Nichterkennen von explosiven Atmosphärenbedingungen und könnte eine Einleitung ungünstiger Gegenmaßnahmen bewirken, die die Integrität des Sicherheitsbehälters gefährden könnten. Um die Annahme derartiger Randbedingungen zu vermeiden und stattdessen eine direkte Ermittlung der realen Atmosphärenbedingungen Im Containment auch bei möglicherweise vorliegenden Überhitzungen oder sonstigen erschwerenden Bedingungen vornehmen zu können, sollte eine Kondensation möglicherweise in der Atmosphäre enthaltenen und somit auch In der Probe mitgeführten Dampfgehalts in der Probenahmeleitung, auch beim Durchqueren von kälteren Sicherheitsbehälterraumbereichen, und somit bereits vor Erreichen der zur Auswertung vorgesehenen Messstelle sicher vermieden werden. Um dies mit besonders hoher betrieblicher Sicherheit und auf passive Weise, d. h. ohne das Erfordernis aktiver Steuerungseingriffe von außen, zu gewährleisten, ist das Probenahmesystem für die Einhaltung eines überhitzten Zustands beim Transport der Probe durch die Probenahmeleitung ausgelegt. Dies wird erreicht durch die konsequente Einhaltung eines Unterdruckzustands in der Probenahmeleitung während des Transports der entnommenen Probe. Der in der Probenahmeleitung bereits vor der Entnahme der eigentlichen Probe bereits aus Transportgründen eingestellte Unterdruck wird dabei auch während des Transports der Probe in der Probenahmeleitung aufrechterhalten, indem die Probenahmeleitung in ihrem Eintrittsbereich geeignet gedrosselt ist.

Um den vorgesehenen Unterdruck beim Transport der Probe durch die Probenahmeleitung auf besonders einfache und zuverlässige Weise aufrecht erhalten zu können, ist die Probenahmeleitung vorteilhafterweise als Kleinleitung mit einer Nennweite von bis zu 15 mm und in besonders vorteilhafter Ausgestaltung als Kapillarleitung mit einer Nennweite von etwa 1 bis 5 mm ausgeführt. Durch eine derartige Ausführung der Probenahmeleitung ist zudem gewährleistet, dass die Volumina der entnommenen Proben besonders gering gehalten werden können, so dass selbst bei vergleichsweise hohen, in der Containment-Atmosphäre freigesetzten Aktivitätsmengen die infolge der Probenahme in den Außenbereich des Reaktorsicherheitsbehälters getragene Gesamtaktivität besonders gering gehalten werden kann. Des Weiteren weist eine derartig dimensionierte Probenahmeleitung auch eine besonders hohe intrinsische Sicherheit gegenüber Beschädigungen auf, da selbst bei unterstelltem vollständigem Abriss der Probenahmeleitung die dadurch entstehende zusätzliche Leckage aus dem Reaktorsicherheitsbehälter an dessen Außenwelt im Vergleich zur auslegungsbedingt ohnehin vorgesehenen Leckage nur unwesentlich ist.

Hierdurch wird es möglich, die bei konventionellen Probenahmesystemen aufgrund der möglichen größeren Leckagequerschnitte vorgeschriebenen doppelten Absperreinrichtungen im Bereich der Sicherheitsbehälterdurchdringung inkl. Einbindung in Reaktorschuztzsysteme generell zu vermeiden. Weiterhin weisen Kapillarleitungen mit einem Durchmesser von z.B. 3 mm und nur 0.5 mm Wandstärke bereits Auslegungsdrücke von mehr als 50 bar auf, was die Sicherheit gegen Versagen bei Sicherheitsbehälter-auslegungsdrücken von z.B. 5 bar grundsätzlich erhöht. Bei Annahme von z. B. 5 bis 10 Probeentnahmen aus dem Sicherheitsbehälter ergibt sich weiterhin eine Einsparung von 10 bis 20 Sicherheitsbehälterabsperrarmaturen. Dies führt einerseits zu einer signifikanten Kostenreduktion und ermöglicht auch - durch die Vermeidung von Armaturen in diesem Bereich - eine optimierte Probenüberhitzung auch im Durchführungsbereich.

Bei der Drosseleinrichtung kann es sich um eine Einzeldrossel oder, beispielsweise um einen größeren Eintrittsdurchmesser zu ermöglichen, auch um eine Mehrstufendrossel oder auch um eine Poröskörperdrosselung handeln. Vorzugsweise weist die Drosseleinrichtung einen freien Strömungsquerschnitt von 0,05 bis 2 mm, vorzugsweise von etwa 0,5 mm auf. Gerade in Kombination mit der vorgesehenen Dimensionierung der Probenahmeleitung ist in einem derartig ausgestalteten Probenahmesystem direkt nach der Probenentnahme eine Druckabsenkung in der Probenahmeleitung auf weniger als etwa 50% des herrschenden Drucks im Reaktorsicherheitsbehälter mit passiven Mitteln gewährleistest. Die vorgesehene direkte Gastrocknung und Überhitzung zur Umgebung durch Drosselung ist damit im kompletten Bereich der Probenahmeleitung innerhalb des Reaktorsicherheitsbehälters sichergestellt. Auch im Durchführungsbereich durch die Außenwand des Sicherheitsbehälters herrschen deutlich günstigere Bedingungen, da selbst bei hohen Dampfpartialdrücken von mehreren bar leicht eine Beheizung von beispielsweise 50 bis 80°C erreicht werden kann - insbesondere durch zusätzliche Beheizung mittels eines Niedrigtemperaturheizelementes - und man somit sicher unter den kritischen Temperaturen für beispielsweise Beton von ca. 80 bis 100 ° C bleiben kann.

Vorteilhafterweise ist der Drosseleinrichtung zudem eine Filtereinheit zugeordnet, so dass Beeinträchtigungen der Funktionsweise selbst bei Auftreten von grobkörnigen Verunreinigungen oder dergleichen ausgeschlossen sind. Die Filtereinheit, die insbesondere auch zur Grobaerosol-Rückhaltung vorgesehen sein kann, enthält dabei vorteilhafterweise ein poröses Filtermaterial, wie beispielsweise Sintermetall oder ein Metallfasergeflecht. Durch die zusätzliche kurzzeitige Rückspülung mittels Druckluft oder Stickstoff, vorteilhafterweise aus Druckflaschen mit Drücken > 10 bis zum Flaschendruck von beispielsweise 100 bar, wird weiterhin eine zuverlässige Freispülung evtl. verunreinigter Drosselquerschnitte erreicht.

Um eine zuverlässige Auswertung der entnommenen Probe und insbesondere eine vergleichsweise genaue Analyse der mitgeführten Gasbestandteile zu ermöglichen, ist die Probentransportleitung außerhalb des Sicherheitsbehälters mit einer Direktbeheizung sowie das der Probenahmeleitung nachgeschaltete Analysesystem vorteilhafterweise mit einem beheizbaren Außengehäuse in der Art eines Wärmeschranks versehen. Dieses ist zweckmäßigerweise derart ausgelegt, dass die Analyse der entnommenen Probe in einem Temperaturbereich von etwa 120 °C, oder bei hohen Überdrücken im Reaktorsicherheitsbehälter bis 160°C, erfolgen kann. Damit ist auch bei der Auswertung der entnommenen Probe eine Auskondensation des Wasserdampfs sicher vermieden, so dass besonders genaue Messwerte für die einzelnen Gasbestandteile der Containment-Atmosphäre ermittelt werden können.

Eine besonders hohe betriebliche Sicherheit und mechanische Stabilität des Probenahmesystem ist erreichbar, indem die Probenahmeleitung vorteilhafterweise in einem Schutzrohr verlegt ist.

Um aus Gründen einer besonders hohen betrieblichen Sicherheit die Anzahl der aktiven Komponenten innerhalb des Reaktorsicherheitsbehälters besonders gering halten zu können, ist das Unterdrucksystem des Probenahmesystems vorteilhafterweise außerhalb des Reaktorsicherheitsbehälters angeordnet. Als Unterdrucksystem kann dabei insbesondere eine außerhalb des Reaktorsicherheitsbehälters angeordnete Pumpenanordnung, beispielsweise eine Membranvakuumpumpe oder eine Strahlpumpe, vorgesehen sein. Alternativ oder zusätzlich kann zur Aufbringung der schnellen Vakuumpulse ein mittels schnellöffnender Ventile zuschaltbarer Vakuumbehälter vorgesehen sein. Bei einer mehrkanaligen Ausführung des Probenahmesystems, also bei einer Parallelschaltung einer Mehrzahl von Probenahmeleitungen, kann anstelle einer zentralen Pumpeneinheit auch für jede Probenahmeleitung eine separate Unterdruckpumpe, insbesondere eine Mikro-Vakuumpumpe, vorgesehen sein.

Das Analysesystem ist vorzugsweise vergleichsweise nah am Reaktorsicherheitsbehälter angeordnet, um die erforderlichen Transportwege gering zu halten. Das Analysesystem kann insbesondere eine Anzahl von Adsorptionssäulen umfassen, wobei in verschiedenen Adsorptionssäulen eine Auftrennung der Gasbestandteile und anschließend eine selektive Messung der Gaskomponenten an Wärmeleitdetektoren am Säulenaustritt erfolgen kann. Dabei ist bereits in geringen Gasvolumina von beispielsweise weniger als 1I eine ganzheitliche Gasanalyse mittels durchströmter Adsorptionssäulen bezüglich des Gehalts von Wasserdampf sowie in parallel geschalteten Adsorptionssäulen des Gehalts von Wasserstoff, Sauerstoff, Kohlendioxid und/oder Kohlenmonoxid, sowie eventuell von Edelgasen, auch unter Störfallbedingungen möglich. Aus diesen Analysenwerten können neben dem Gefährdungspotential der Sicherheitsbehälteratmosphäre auch konkrete Informationen über einen möglichen Schadenszustand des Reaktorkernes und dessen Lage - z.B. bei CO- Nachweis - gewonnen werden. Alternativ oder als diversitäre Messung zur Erhöhung der Sicherheit kann die Bestimmung der Wasserstoff-Konzentration einfacherweise im Wärmeschrank mittels eines Wärmeleitdedektors sowie die Bestimmung des Dampfanteiles mittels kapazitiver Polymere, jeweils direkt im Messgas, erfolgen. Die entsprechenden strahlen- und temperaturempfindlichen Mikroprozesselektroniken der Auswerteeinheit werden getrennt außerhalb des beheizten Schrankes abgeschirmt angebracht.

Das System wird dabei vorzugsweise so betrieben, dass die Messensensoren jeweils nach der erfolgter Analyse mit inaktiven Gasen gespült werden und somit die Strahlenbelastung auch im Analysatorbereich gegenüber kontinuierlicher Analyse deutlich gesenkt wird. Die Steuerung des Systems und der Einrichtungen wird vorteilhafterweise mittels frei programmierbarer Digitalsteuerung durchgeführt, so dass z. B. je nach realer Einbausituation in der Anlage - unter Berücksichtigung der unterschiedlichen Transportzeiten- die entsprechenden Vakuumimpulse durch Änderung der Parameter vor Ort angepasst werden können. Durch eine qualitativ hochwertige Isolierung von < 100 W/m2 werden die thermischen Verluste im Bereich Rohrleitung und Schrank auf < 5 kW Dauerleistung minimiert, so dass die elektrische Versorgung - auch bei Strom-ausfällen - permanent oder kurzfristig zuschaltbar günstig über ein Batterienetz oder separate Notstromdiesel sichergestellt werden kann.

Um eine gezielte Zuführung der entnommenen Probe in das Analysesystem zu ermöglichen, ist vorzugsweise in die Probenahmeleitung vor Eintritt oder bei Eintritt in das Analysesystem ein Probenisolationsbehälter geschaltet. Diesem kann weiterhin ein Pufferbehälter oder Rohrleitungspuffer vorgeschaltet sein, dessen Volumen in zweckmäßiger Ausgestaltung etwa um den Faktor 2 bis 10 größer ist als das Volumen des Probenisolationsbehälters. Durch diese Maßnahme wird sichergestellt, dass nach der verlustfreien Unterdruckbetriebsphase der Entnahmeleitungen bis Containmentdurchführung in der sich anschließenden Druckaufbauphase durch die dann mögliche Unterschreitung des Taupunktes in der Kapillarleitung innerhalb des Sicherheitsbehälters kein verändertes Messgas bis in das Messgasvolumen (Probenisolationsbehälter) transportiert werden kann. Durch anschließendes Rückspülen mit trockenem Gas, z. B. Stickstoff, erfolgt die Trocknung dieser Abschnitte vor der nächsten Probenahme. Alternativ kann die Messgaskompression auch durch Gaseinspeisung eingeleitet werden, wobei aufgrund des vorgeschalteten Volumens und der bei geringen Abmessungen auftretenden Kolbenströmung in der Rohrleitung wiederum ein Eintrag von verändertem Messgas in der Probenbereitstellungsbehälter vermieden wird.

Um die auslegungsgemäß vorgesehene Aufrechterhaltung der Überhitzung der entnommenen Probe während ihres Transports durch die Probenahmeleitung sicher zu stellen, ist die Probenahmeleitung vorteilhafterweise im Bereich außerhalb des Reaktorsicherheitsbehälters beheizbar ausgestaltet. Damit kann ohne das Erfordernis der Anbringung aktiver Komponenten im Innenraum des Reaktorsicherheitsbehälters sichergestellt werden, dass bedarfsweise durch gezielte Beheizung einzelner Bereiche der Probenahmeleitung die Auskondensation von Wasserdampf auch bei vergleichsweise langen Transportwegen vermieden ist.

Um die bei einer Probenahme möglicherweise in den Außenbereich freigesetzten Aktivitäten besonders gering zu halten, ist an die Probenahmeleitung in weiterer vorteilhafter Ausgestaltung eine in den Reaktorsicherheitsbehälter mündende Rückführleitung angeschlossen. Dabei kann insbesondere eine Rückförderung der entnommenen Aktivitäten mittels einer Kompressoreinheit und/oder durch Anordnung einer Edelgasverzögerungsstrecke, beispielsweise auf Basis von Aktivkohle oder Zeolith, erfolgen. Dies ist mit besonders einfachen Mitteln erreichbar, indem vorzugsweise die Förderung und Vakuumerzeugung mittels eines Gasstrahlers oder zumindest temporär mit aus Gasflaschen entnommenem Druckgas betrieben wird.

Bezüglich des Verfahrens zur Gewinnung einer Probe der genannten Art wird die weiter oben genannte Aufgabe gelöst durch die Merkmale des Anspruchs 17.

Dabei ist vorgesehen, dass in einer Probenahmeleitung ein Unterdruck im Vergleich zum im Reaktorsicherheitsbehälter herrschenden Druck erzeugt wird, wobei nach dem Einströmen der Probe in die Probenahmeleitung der Druck in der Probenahmeleitung auf maximal etwa 60 % des im Reaktorsicherheitsbehälter herrschenden Drucks begrenzt wird. Dies wird vorteilhafterweise dadurch gewährleistet, dass die Einströmung der Probe in die Probenahmeleitung und/oder die Einströmung von Atmosphärenbestandteilen in die Probenahmeleitung gedrosselt wird.

Das beschriebene Konzept zur Probenahme und anschließenden Analyse arbeitet hinsichtlich der erreichbaren Genauigkeit und Zuverlässigkeit im Wesentlichen unabhängig von der im Reaktorsicherheitsbehälter aktuell vorliegenden Sauerstoff-Konzentration. Eine weitere Erhöhung Zuverlässigkeit und somit der betrieblichen Sicherheit ist vorteilhafterweise erreichbar durch eine Kombination dieses Verfahrens mit einem so genannten Wärmetönungsverfahren zur Messung der Wasserstoff-Konzentration im Reaktorsicherheitsbehälter, welches abhängig von der Sauerstoff-Konzentration arbeitet.

Hierbei erfolgt vorzugsweise zusätzlich an einigen Stellen im Reaktorsicherheitsbehälter in der Art einer diversitären Redundanz die Messung der Wasserstoff-Konzentration nach dem Wärmetönungsprinzip. Die dafür vorgesehenen Messstellen der beiden Verfahren sind vorteilhafterweise in den gleichen Raumbereichen des Reaktorsicherheitsbehälters angeordnet, so dass besonders in der frühen Phase eines möglichen Unfallverlaufes durch einen Vergleich der von beiden Verfahren gelieferten Messwerte miteinander die Brenngaskonzentration, die Sauerstoff- und die tatsächliche Wasserstoff-Konzentration vergleichsweise genau bestimmt werden können.

Beim Wärmetönungsverfahren werden in einem Sensorkopf, der direkt in der Atmosphäre des Reaktorsicherheitsbehälters installiert ist, ein katalytisch aktives und ein nicht katalytisch wirkendes beheiztes Filament untergebracht. Bei Vorliegen von Wasserstoff in der umgebenden Atmosphäre erfolgt, je nach vorliegender Sauerstoff-Konzentration, eine Oxidation am katalytisch aktiven Filament, welches über Kabel mit einer außerhalb des Reaktorsicherheitsbehälters angeordneten Elektronik verbunden ist.

Die daraus infolge der einsetzenden Temperaturerhöhung resultierende elektrische Widerstandsänderung wird durch eine Brückenschaltung elektrisch kompensiert. Der Kompensationsstrom ist ein direktes Maß für die erfolgte Wasserstoff-Oxidation und kann als Wasserstoff-Meßsignal oder Brenngaskonzentration ausgegeben werden.

Die dabei gewonnenen Signale werden zweckmäßigerweise zusätzlich in einer von beiden Analyseverfahren genutzten Steuerungs- und Auswerteeinheit aufbereitet.

Die durch Messung mittels dem vorstehend beschriebenen Verfahren durch Wärmeleitfähigkeit ermittelte Wasserstoff-Konzentration entspricht der tatsächlichen Konzentration, unabhängig von der aktuellen Sauerstoff- Konzentration. Durch Vergleich der mit beiden Verfahren ermittelten Wasserstoff-Konzentrationswerte lässt sich somit die tatsächliche Wasserstoff-Konzentration (Wärmeleitfähigkeit) sowie zusätzlich, bei Vorliegen von Sauerstoff-Überschuß über die Wärmetönung, die Wasserstoff-Konzentration redundant bestimmen.

In den in möglichen Unfallsituationen sicherheitstechnisch relevanten, wahrscheinlich vorliegenden Fällen vergleichsweise hoher Wasserstoff-Konzentrationen bei gleichzeitig reduzierten Sauerstoff-Konzentrationen kann nach dem Wärmeleitprinzip die maximale Wasserstoff-Konzentration, nach dem Sensor-Wärmetönungsverfahren die Brenngaskonzentration und zusätzlich die Sauerstoff-Konzentration ermittelt werden.

Durch die Überwachung der gewonnenen Messwerte mittels einer geeigneten Rechnerschaltung aus der Probenahme-Analyse und dem permanenten Vergleich mit den Messwerten des Sensor/Kabel-Verfahrens können zudem die Wasserstoff-Freisetzungsraten sowie aufgrund der definierten Sauerstoff-Menge im Reaktorsicherheitsbehälter zusätzlich die Wasserstoff-Oxidationsraten ermittelt und bilanziert werden. Hier-durch können neben einem aktuellen Gefährdungspotential für die Anlage insbesondere auch wichtige Rückschlüsse auf den Störfallverlauf, beispielsweise ob die Brennstaboxidation gestoppt werden konnte, gezogen werden, so dass die gezielte Einleitung entsprechender Gegenmaßnahmen ermöglicht ist.
Die genannten Verfahren werden vorteilhafterweise insbesondere in der transienten frühen Unfallphase angewandt, da im weiteren Unfallverlauf der Sauerstoff im Sicherheitsbehälter reagiert. Die Kabel des Sensor/Kabel-Verfahrens können daher vorteil-hafterweise im Hinblick auf den Herstellungs- und Montageaufwand als Kunststoffkabel unter Verzicht auf ein vollkeramisches Kabeldesign ausgeführt sein, wobei eine Auslegung auf eine Funktionsdauer bei mittlerer Strahlenbelastung von bis zu 24h als ausreichend angesehen wird. Bei Fortschreiten des Unfalls wird, aufgrund der dauerhaft hohen Strahlenbelastung im Reaktorsicherheitsbehälter, ein Kabelausfall toleriert, der ebenfalls von der Elektronik erkannt wird, so daß anschließend nur noch das Probenahme- und -analyseverfahren zur Messung herangezogen wird.

Die mit der Erfindung erzielten Vorteile bestehen insbesondere darin, dass durch die Einstellung eines geeignet gewählten Unterdrucks in der Probenahmeleitung und dessen Aufrechterhaltung durch Drosselung bei der Einströmung der Probe in die Probenahmeleitung auch während des Transports der Probe von der Entnahmestelle zum außerhalb des Reaktorsicherheitsbehälters angeordneten Analysesystem konsequent ein überhitzter Zustand der entnommenen Probe eingehalten werden kann. Damit ist die zu einer möglichen Verfälschung von Analyseergebnissen führende Auskondensation von Wasserdampf während des Transports der entnommenen Probe ausgeschlossen. Die Probe kann somit in einem Zustand analysiert werden, in dem sie die tatsächlichen Verhältnisse innerhalb des Reaktorsicherheitsbehälters besonders genau wiedergibt. Damit sind besonders zuverlässige Messergebnisse über den aktuellen Ist-Zustand der Containment-Atmosphäre ermittelbar, ohne dass ein Rückgriff auf verallgemeinernde Angaben oder Schätzwerte erforderlich wäre. Durch die somit erreichbare besonders genaue Istwert-Ermittlung für die Containment-Atmosphäre ist eine besonders bedarfsgerechte Führung und Steuerung von Gegenmaßnahmen und somit eine besonders sichere Betriebsführung auch beim Störfallmanagement ermöglicht.

Durch die geeignet gewählte Dimensionierung der Probenahmeleitung und der weiteren Komponenten ist zudem die bei der Probenahme erfolgende Entnahme an Aktivität selbst bei vergleichsweise gravierenden Unfallsituationen besonders gering gehalten, so dass eine Aktivitätsfreisetzung an die Umwelt besonders gering gehalten werden kann. Durch eine qualitativ hochwertige Isolierung von < 100 W/m2 werden die thermischen Verluste im Bereich Rohrleitung und Schrank auf < 5 kW Dauerleistung minimiert, so daß die elektrische Versorgung - auch in station black Situationen - günstig über ein Batterienetz oder separate Notstromdiesel, permanent oder kurzfristig sichergestellt werden kann. In der frühen Phase eines möglichen Unfallverlaufes kann eine permanente Wasserstoff-Konzentrationsmessung durch eine günstige Kombination mit zusätzlich im Reaktorsicherheitsbehälter angebrachten Wasserstoffsensoren und des hier beschriebenen Verfahrens erreicht werden. Hierbei erfolgt zusätzlich an wenigen Stellen im Reaktorsicherheitsbehälter die Messung der Wasserstoff-Konzentration nach dem Wärmetönungsprinzip. Hierbei werden im Sensorkopf ein katalytisch aktives und ein nicht katalytisch wirkendes beheiztes Filament untergebracht. Bei Wasserstoff-Anfall erfolgt die Oxydation am katalytisch aktiven Filament, welches über Kabel mit einer außerhalb des Reaktorsicherheitsbehälters angeordneten Elektronik verbunden ist. Die resultierende elektrische Widerstandsänderung wird durch eine Brükkenschaltung elektrisch kompensiert. Die gewonnenen Signale werden zusätzlich in einer von beiden Verfahren genutzten Steuerungs- und Auswerteeinheit aufbereitet. Hiermit kann kurzfristig ein permanentes Wasserstoff-Signal erzeugt werden, und ab Verfügbarkeit der vorliegenden Analyse und des durch Wärmeleitfähigkeit gewonnenen Wasserstoff-Messwertes kann eine Aussage zum Sauerstoffgehalt der Atmosphäre gewonnen werden.

Durch die Überwachung der gewonnen Messwerte und Vergleich mit Sensor/Kabel-Verfahren kann der Ausfallzeitpunkt aufgrund der extremen Strahlenbelastung im Kabelbereich zusätzlich überwacht werden.

Ein Ausführungsbeispiel der Erfindung wird anhand einer Zeichnung näher erläutert. Darin zeigen:
- Figur 1: ein Probenahmesystem,
- Figur 2: eine alternative Ausführungsform eines Probenahmesystems, und
- Figur 3: eine Drosseleinrichtung zur Anwendung in einem Probenahmesystem nach Figur 1 oder Figur 2.

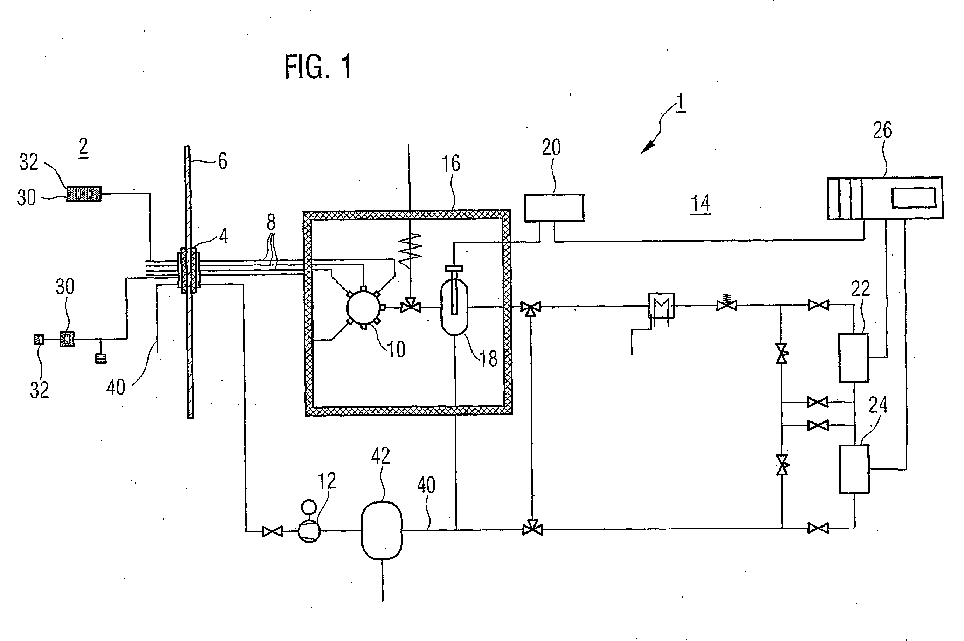

Gleiche Teile sind in allen Figuren mit denselben Bezugszeichen versehen.
Das Probenahmesystem 1 gemäß Figur 1 ist zur Gewinnung einer Probe aus der Atmosphäre in einem Containment oder Reaktorsicherheitsbehälter 2 einer nicht weiter dargestellten kerntechnischen Anlage vorgesehen. Dazu umfasst das Probenahmesystem 1 eine Mehrzahl von in den Reaktorsicherheitsbehälter 2 mündenden, über eine Durchführung 4 durch dessen Außenwand 6 geführten Probenahmeleitungen 8. Diese sind über einen Ventilblock 10, über den eine selektive und spezifische Anwahl einer beliebigen Probenahmeleitung 8 erfolgen kann, mit einem Unterdrucksystem 12 und mit einem Analysesystem 14 verbunden. Das Analysesystem 14 umfasst dabei ein in der Art eines Wärmeschranks beheizbar ausgeführtes Außengehäuse 16, in dem zusätzlich zum Ventilblock 10 ein ummantelter Gasanalysator 18 angeordnet ist. Der Gasanalysator 18, z.B. ausgeführt als kapazitiver Sensor, ist mit einer ersten Messelektronik 20 zur Ermittlung des Wasserdampfgehalts, mit einer zweiten Messstelle 22 zur Ermittlung eines Wasserstoffanteils -vorzugsweise nach dem Wärmeleitprinzip - und mit einer dritten Messstelle 24 zur Ermittlung eines Sauerstoffanteils verbunden. Die Messstellen 20, 22, 24 sind ausgangsseitig mit einer zentralen Auswerte-Elektronikeinheit 26 verbunden, die auch die komplette Systemsteuerung übernimmt und ggf. Vergleich mit diversitären Sensorsignal. In einer nicht dargestellten Variante kann der Kühler entfallen und die Messung 22 und 24 auch im Wärmeschrank 16 angeordnet werden.

Das Probenahmesystem 1 ist für die Gewinnung besonders genauer und zuverlässiger Messergebnisse für die Gasanteile der Atmosphäre innerhalb des Sicherheitsbehälters 2 ausgelegt. Dazu ist vorgesehen, bei der Überführung der Probe vom Innenraum des Reaktorsicherheitsbehälters 2 in den Gasseparator 18 gezielt eine Auskondensation von mitgeführtem Wasserdampf zu vermeiden, so dass der mitgeführte Wasserdampf qualitativ und auch quantitativ in der nachfolgenden Auswertung erfasst und berücksichtigt werden kann. Um dies zu ermöglichen, ist das Probenahmesystem 1 dafür ausgelegt, die Probe in überhitztem Zustand in den Gasseparator 18 des Analysesystems 14 zu überführen. Der überhitzte Zustand wird dabei mit passiven Mitteln, also ohne die Notwendigkeit eines aktiven äußeren Eingriffs, eingestellt und aufrecht erhalten, indem beim Transport der Probe in der Probenahmeleitung 8 ein geeignet gewählter Unterdruck eingestellt und aufrecht erhalten wird. Dazu sind die Probenahmeleitungen 8 an ihrem in den Reaktorsicherheitsbehälter 2 mündenden Ende jeweils mit einer Drosseleinrichtung 30 versehen.

Die Drosseleinrichtung 30, der zur Vermeidung einer Verblockung und auch zur Grobaerosolzurückhaltung ein Filter 32, beispielsweise bestehend aus porösem Filtermaterial wie beispielsweise Sintermetall oder einem Metallfasergeflecht, vorgeschaltet ist, ist dabei gezielt dafür ausgelegt, in der jeweiligen Probenahmeleitung 8 auch bei Einströmen von Atmosphäre aus dem Innenraum des Reaktorsicherheitsbehälters 2 einen Unterdruck von insbesondere weniger als etwa 50% des Containment-Drucks aufrecht zu erhalten. Um dies mit vergleichsweise einfachen Mitteln zu ermöglichen, ist die jeweilige Probenahmeleitung 8 einerseits als Kapillarleitung mit einer vergleichsweise geringen Nennweite von etwa 3 mm ausgeführt. Andererseits ist die jeweils vorgeschaltete Drosseleinrichtung 30 geeignet dimensioniert und mit einem freien Strömungsquerschnitt von etwa 0,5 mm ausgelegt. Der Probentransport erfolgt aufgrund des hohen Unterdruckes in den Kapillarleitungen vorzugsweise mit > 5 m/s bis 50m/s, so dass geringe Transportzeiten realisiert werden können.

Zusätzlich sind die Probenahmeleitungen 8 im Bereich außerhalb der Außenwand 6 des Reaktorsicherheitsbehälters 2 beheizbar ausgeführt, Die Durchdringung des Reaktorsicherheitsbehälters ist mit Niedrigtemperaturelementen auf < 80°C beheizt. Somit ist gewährleistet, dass auch bei vergleichsweise langer Leitungsführung eine Aufrechterhaltung des überhitzten Zustands der entnommenen Probe bis zum Eintreffen im Analysesystem 14 gewährleistet ist.
Zur Rückführung möglicherweise gemeinsam mit der Probe entnommener Aktivitäten in den Innenraum des Reaktorsicherheitsbehälters 2 ist die jeweilige Probenahmeleitung 8 mit einer in den Reaktorsicherheitsbehälter 2 mündenden Rückführleitung 40 verbunden. Die Rückführleitung 40, in die zur bedarfsweisen Pufferung ein Rückförderbehälter 42 geschaltet ist, ist dabei mit der als Unterdrucksystem 12 vorgesehenen Vakuumpumpe versehen, so dass diese zusätzlich zur Evakuierung der jeweiligen Probenahmeleitung 8 auch zur Rückförderung in den Reaktorsicherheitsbehälter 2 eingesetzt werden kann.

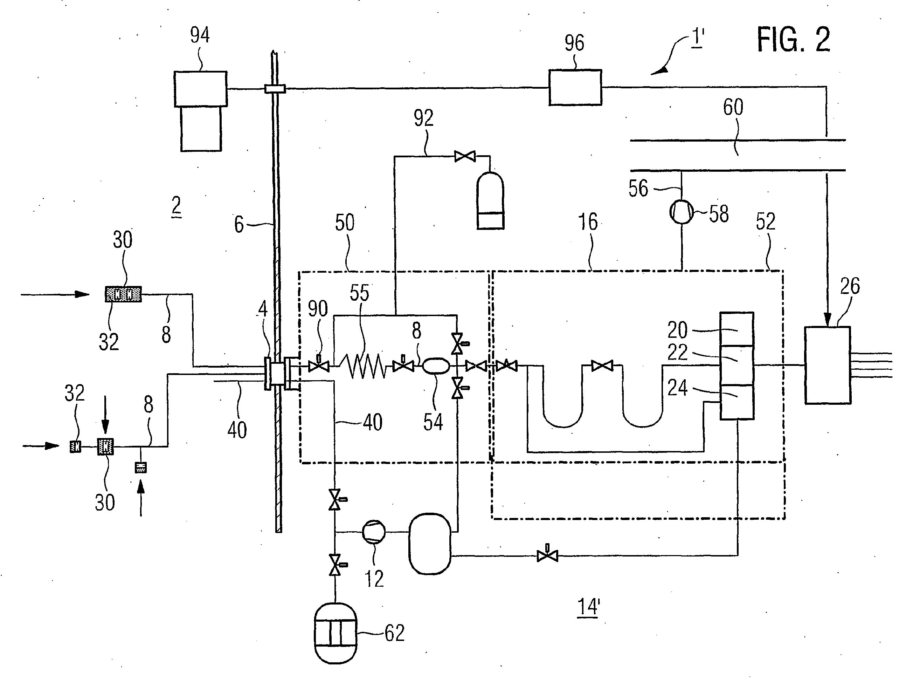

Im Ausführungsbeispiel nach Figur 2 weist das Probenahmesystem 1' ein Analysesystem 14' auf, das im Wesentlichen modular aufgebaut ist. Das Analysesystem 14' umfasst dabei ein Probenahmemodul 50 sowie ein Messmodul 52, das in dem als Wärmeschrank ausgeführten beheizbaren Außengehäuse 16 angeordnet ist. In diesem Ausführungsbeispiel ist im Probenahmemodul 50 ein Probenisolationsbehälter 54 angeordnet, in dem die über eine Probenahmeleitung 8 aus dem Innenraum des Reaktorsicherheitsbehälters 2 entnommene Probe zwischengespeichert, direkt analysiert oder für eine nachfolgende Auswertung bereit gehalten werden kann. Das Probennahmemodul 50 umfasst weiterhin gegebenenfalls erforderliche Messsonden, Mikromehrportventile, Mikroabsperrventile, schnellöffnende Vakuumventile und Drosseln und/oder Druckreduzierventile.

Durch ein dem Probeisolationsbehälter 54 in der Probenahmeleitung 8 vorgeschaltetes Zusatzvolumen 55 kann sichergestellt werden, dass nach dem Unterdruckbetrieb in der sich anschließenden Druckaufbauphase durch die dann mögliche Unterschreitung des Taupunktes in der Kapillarleitung innerhalb des Reaktorsicherheitsbehälters 2 verändertes Messgas nicht in das Messgasvolumen transportiert werden kann. Alternativ kann durch Schließen des in die Probenahmeleitung 8 geschalteten Ventils 90 und einem Druckaufbau über eine Gaseinspeisung 92 und Durchströmen des Zusatzvolumens 55 nach der gleichen Methode ein Druckaufbau im Probenisolationsbehälter 54 ohne Messgasverfälschung erreicht werden. Durch das Rückspülen mit trockenem Gas, z. B. Stickstoff, erfolgt eine Trocknung dieser Abschnitte vor der nächsten Probenahme. Alternativ kann die Messgaskompression auch durch die Gaseinspeisung 92 und das Schließen des Ventils 90 eingeleitet werden, wobei aufgrund des vorgeschalteten Zusatzvolumens 55 und der Kolbenströmung in der Rohrleitung wiederum ein Eintrag von verändertem Messgas in den Probenbereitstellungsbehälter vermieden wird.
Das Zusatzvolumen 55 kann dabei insbesondere auch als Puffervolumen dienen und ist etwa um den Faktor zwei bis fünf größer als das Innenvolumen des Probenisolationsbehälters 54.

Im nachgeschalteten Messmodul 52 sind Probendosierkomponenten sowie die zur eigentlichen Messung erforderlichen Adsorptionssäulen und die Messstellen und Sensoren 20, 22, 24 enthalten. Das Messmodul 52 ist über eine Abführleitung 56, in die ein Unterdruckventilator 58 geschaltet ist, mit einem Abluftsystem 60 verbunden.

Im Probenahmesystem 1' ist zudem zur Gewährleistung einer besonders gering gehaltenen Aktivitätsfreisetzung die Rückführleitung 40 mit einer Edelgasverzögerungsstrecke 62, insbesondere auf Aktivkohle- oder Zeolith-Basis, verbunden.
Wie ebenfalls in Fig.2 gezeigt, ist zusätzlich in Kombination oder diversitär zum Probenahmesystem eine Wasserstoff-Messung vorgesehen. Diese umfasst eine Anzahl von im Reaktorsicherheitsbehälter 2 angebrachten Wasserstoffsensoren 94, die datenseitig an eine gemeinsame außerhalb des Reaktorsicherheitsbehälters 2 angeordnete Auswerteeinheit 96 angeschlossen sind. Die in dieser gewonnenen Signale werden zusätzlich der von beiden Verfahren genutzten Steuerung- und Auswerte-Elektronikeinheit 26 zugeführt.

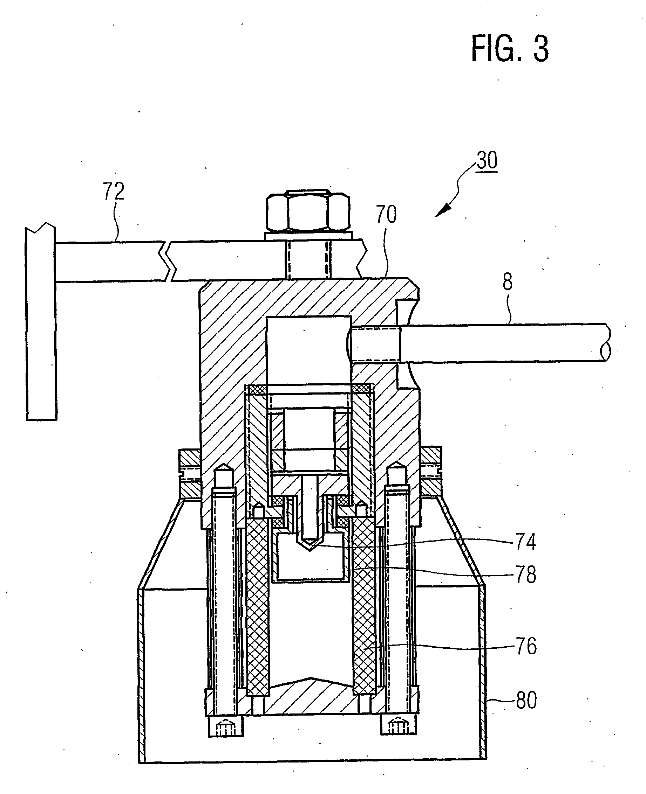

Ein Ausführungsbeispiel für die der jeweiligen Probenahmeleitung vorgeschaltete Drosseleinrichtung 30 ist in Figur 3 im Querschnitt vergrößert dargestellt. Die Drosseleinrichtung 30 umfasst einen Grundkörper 70, der über einen geeignet dimensionierten und gewählten Wandhalter 72 an einem Wandelement des Reaktorsicherheitsbehälters 2 befestigt ist. Der Grundkörper 70 ist weiterhin mit dem Mündungsende der Probenahmeleitung 8 verbunden.

Um ein gedrosseltes Einströmen von Atmosphärengas in die Probenahmeleitung 8 zu gewährleisten, umfasst die Drosseleinrichtung 30 einen Drosselkörper 74, der im Vergleich zur Nennweite der als Kapillarleitung ausgeführten Probenahmeleitung 8 von etwa 3 mm einen geringen freien Strömungsquerschnitt von etwa 0,5 mm aufweist. Der Einströmbereich des Drosselkörpers 74 ist von einer im Wesentlichen zylindrisch ausgebildeten, in der Art eines Grobdemisters vorgesehenen Tröpfchen- oder Feststoffabscheiders 76 umgeben. Innerhalb des Abscheiders 76 ist zur Bildung einer Filtereinrichtung ein Filterkörper 78 aus Sintermetall oder einem Metallfasergewebe angeordnet. Die aus diesen Komponenten gebildete Anordnung ist von einer als Sprayschutz vorgesehenen Ummantelung 80 umgeben.

### Bezugszeichenliste

- 1, 1': Probenahmesystem
- 2: Reaktorsicherheitsbehälter
- 4: Durchführung
- 6: Außenwand
- 8: Probenahmeleitungen
- 10: Ventilblock
- 12: Unterdrucksystem
- 14, 14': Analysesystem
- 16: Außengehäuse
- 18: Gasanalysator / Gasseparator in Probenisolationsbehälter
- 20, 22, 24: Messstelle
- 26: Auswerte-Elektronikeinheit und Prozesssteuerung
- 30: Drosseleinrichtung
- 32: Filter
- 40: Rückführleitung
- 42: Rückförderbehälter
- 50: Probenentahmemodul
- 52: Messmodul
- 54: Probeisolationsbehälter
- 55: Zusatzvolumen
- 56: Abführleitung
- 58: Unterdruckventilator
- 60: Abluftsystem
- 62: Edelgasverzögerungsstrecke
- 70: Grundkörper
- 72: Wandhalter
- 74: Drosselkörper
- 76: Tröpfchen- oder Feststoffabscheider
- 78: Filterkörper
- 80: Ummantelung
- 90: Ventil
- 92: Gaseinspeisung
- 94: Wasserstoffsensoren
- 96: Auswerteeinheit

## Patentansprüche

1. Kerntechnische Anlage mit einem Reaktorsicherheitsbehälter (2) und mit einem zur Gewinnung einer Probe aus der Atmosphäre im Reaktorsicherheitsbehälter (2) ausgelegten Probenahmesystem (1, 1'), wobei das Probenahmesystem (1, 1') eine mit einem Unterdrucksystem (12) und mit einem Analysesystem (14) verbundene, in den Reaktorsicherheitsbehälter (2) mündende Probenahmeleitung (8) umfasst, **dadurch gekennzeichnet, dass**
der Probenahmeleitung (8) gasseitig bei Verbindung derselben mit der Atmosphäre im Reaktorsicherheitsbehälter (2) eine Drosseleinrichtung (30) vorgeschaltet ist.

2. Kerntechnische Anlage nach Anspruch 1, wobei die Probenahmeleitung (8) als Kleinleitung mit einer Nennweite von bis zu 15 mm, vorzugsweise als Kapillarleitung mit einer Nennweite von etwa 1 bis 5mm, ausgeführt ist.

3. Kerntechnische Anlage nach Anspruch 1 oder 2, wobei die Drosseleinrichtung (30) einen freien Strömungsquerschnitt von 0,05 bis 2 mm, vorzugsweise von etwa 0,5 mm, aufweist.

4. Kerntechnische Anlage nach einem der Ansprüche 1 bis 3, wobei der Drosseleinrichtung (30) eine Filtereinheit (32) zugeordnet ist.

5. Kerntechnische Anlage nach einem der Ansprüche 1 bis 4, wobei das Analysesystem (14) mit einem beheizbaren Außengehäuse (16) versehen ist.

6. Kerntechnische Anlage nach einem der Ansprüche 1 bis 5, wobei eine Außenisolierung im Bereich des Analysesystems (14) die thermischen Verluste auf weniger als 100 W/m², vorzugsweise auf weniger als 50 W/m², begrenzt.

7. Kerntechnische Anlage nach einem der Ansprüche 1 bis 6, wobei eine elektrische Versorgung vorgesehen ist, die zur Absicherung gegen Stromausfälle ein Batterienetz und / oder separate Notstromdieselaggregate umfasst.

8. Kerntechnische Anlage nach einem der Ansprüche 1 bis 7, wobei dem Probenahmesystem (1, 1') zur Steuerung und Regelung eine freiprogrammierbare digitale Steuerungseinheit zugeordnet ist.

9. Kerntechnische Anlage nach einem der Ansprüche 1 bis 8, wobei die Probenahmeleitu ng (8) in einem Schutzrohr verlegt ist.

10. Kerntechnische Anlage nach einem der Ansprüche 1 bis 9, wobei das Unterdrucksystem (12) außerhalb des Reaktorsicherheitsbehälters (2) angeordnet ist.

11. Kerntechnische Anlage nach einem der Ansprüche 1 bis 10, wobei in die Probenahmeleitung (8) vor Eintritt in das Analysesystem (14) ein Probenisolationsbehälter (54) geschaltet ist.

12. Kerntechnische Anlage nach Anspruch 11, wobei der Probenisolationsbehälter (54) ein um den Faktor zwei bis fünf kleineres Innenvolumen als ein vorgeschaltetes Puffervolumen aufweist.

13. Kerntechnische Anlage nach einem der Ansprüche 1 bis12, wobei die Probenahmeleitung (8) in einem Bereich außerhalb des Reaktorsicherheitsbehälters (2) beheizbar ist.

14. Kerntechnische Anlage nach einem der Ansprüche 1 bis 13, wobei das Analysesystem (14) zur Gasanalyse auf die Bestandteile Wasserstoff und/oder Dampfgehalt und/oder Kohlenmonoxid eine Anzahl von kapazitiven Polymersensoren und/oder Wärmeleitdedektoren umfasst.

15. Kerntechnische Anlage nach einem der Ansprüche 1 bis 14, wobei an die Probenahmeleitung (8) eine in den Reaktorsicherheitsbehälter (2) mündende Rückführleitung (40) angeschlossen ist.

16. Kerntechnische Anlage nach einem der Ansprüche 1 bis 15, mit einer Anzahl von nach dem Wärmetönungsprinzip ausgelegten Wasserstoff-Sensoren (94), die innerhalb des Reaktorsicherheitsbehälters (2) angeordnet, datenseitig mit einer externen Auswerteeinheit 96 und über diese mit einer gemeinsamen Auswerte-Elektronikeinheit 26 für das Probenahmesystem (1, 1') verbunden sind.

17. Verfahren zur Gewinnung einer Probe aus der Atmosphäre in einem Reaktorsicherheitsbehälter (2) einer kerntechnischen Anlage, bei dem in einer Probenahmeleitung (8) ein Unterdruck im Vergleich zum im Reaktorsicherheitsbehälter (2) herrschenden Druck erzeugt wird,
**dadurch gekennzeichnet, dass**
nach dem Einströmen der Probe in die Probenahmeleitung (8) der Druck in der Probenahmeleitung (8) auf maximal etwa 60% des im Reaktorsicherheitsbehälter (2) herrschenden Drucks begrenzt wird.

18. Verfahren nach Anspruch 17, bei dem der Unterdruck durch schnellöffnende Ventile und ein Saugvolumen aus einem Unterdruck/Rückförderbehälter spontan aufgebracht wird.

19. Verfahren nach Anspruch 17 oder 18, bei dem nach erfolgter Probenahme ein Druckwechsel mit Rückspülung von Analysegeräten und Entnahmeleitung durchgeführt wird.

20. Verfahren nach einem der Ansprüche 17 bis 19, bei dem das Probenvolumen per Einzelanalyse auf <1 I und/oder auf einen Aktivitätsinhalt von < 10¹⁰ Bq begrenzt wird.

21. Verfahren nach einem der Ansprüche 17 bis 20 bei dem die Einströmung der Probe in die Probenahmeleitung (8) gedrosselt wird.

22. Verfahren nach einem der Ansprüche 17 bis 21, bei dem bei Dampfpartialdrücken von mehreren bar an den Entnahmestellen durch Druckabsenkung in der Analyseeinheit auf vorzugsweise 1bar eine Unterschreitung des Taupunkts im Messgas vermieden wird.

23. Verfahren nach einem der Ansprüche 17 bis 22, bei dem ein zusätzlicher Messwert für die Wasserstoff-Konzentration im Reaktorsicherheitsbehälter (2) nach dem Wärmetönungsverfahren ermittelt wird.

## Claims

1. A nuclear facility having a reactor containment (2) and having a sampling system (1, 1') designed for extracting a sample from the atmosphere inside the reactor containment (2), the sampling system (1, 1') comprising a sampling line (8) connected with a vacuum system (12) and with an analyzing system (14) and opening into the reactor containment (2),
**characterized in that**
a throttle device (30) is connected upstream of the sampling line (8) on the gas side when the sampling line (8) is connected with the atmosphere inside the reactor containment (2).

2. The nuclear facility of claim 1, wherein the sampling line (8) is designed as a small line with a nominal diameter of up to 15 mm, preferably as a capillary line with a nominal diameter of approx. 1 to 5 mm.

3. The nuclear facility of claim 1 or 2, wherein the throttle device (30) has a free cross-section of flow of 0.05 to 2 mm, preferably of approx. 0.5 mm.

4. The nuclear facility of any of claims 1 to 3, wherein a filter unit (32) is associated with the throttle device (30).

5. The nuclear facility of any of claims 1 to 4, wherein the analyzing system (14) is provided with a heatable external housing (16).

6. The nuclear facility of any of claims 1 to 5, wherein an external insulation in the region of the analyzing system (14) limits the thermal losses to less than 100 W/m², preferably to less than 50 W/m².

7. The nuclear facility of any of claims 1 to 6, wherein an electric supply is provided, which comprises a battery network and/or separate emergency-power diesel sets, as a protection against power failure.

8. The nuclear facility of any of claims 1 to 7, wherein a freely programmable digital control unit is associated with the sampling system (1, 1'), for the purpose of control and regulation.

9. The nuclear facility of any of claims 1 to 8, wherein the sampling line (8) is laid in a protecting tube.

10. The nuclear facility of any of claims 1 to 9, wherein the vacuum system (12) is arranged outside the reactor containment (2).

11. The nuclear facility of any of claims 1 to 10, wherein a sample insulating container (54) is connected into the sampling line (8) before it enters the analyzing system (14).

12. The nuclear facility of claim 11, wherein the inner volume of the sample insulating container (54) is two to five times smaller than an upstream buffer volume.

13. The nuclear facility of any of claims 1 to 12, wherein the sampling line (8) is heatable in a region outside the reactor containment (2).

14. The nuclear facility of any of claims 1 to 13, wherein the analyzing system (14) comprises a number of capacitive polymer sensors and/or heat-conduction detectors for analyzing the gas for the components hydrogen and/or vapor content and/or carbon monoxide.

15. The nuclear facility of any of claims 1 to 14, wherein a recirculation line (40) opening into the reactor containment (2) is connected to the sampling line (8).

16. The nuclear facility of any of claims 1 to 15, having a number of hydrogen sensors (94) designed according to the heat-tone principle, which are arranged inside the reactor containment (2) and are connected on the data side with an external evaluation unit (96) and via the latter, with a common electronic evaluation unit (26) for the sampling system (1, 1').

17. A method for extracting a sample from the atmosphere inside a reactor containment (2) of a nuclear facility, wherein a lower pressure than the pressure prevailing in the reactor containment (2) is generated in a sampling line (8), **characterized in that**,
after the sample has flowed into the sampling line (8), the pressure in the sampling line (8) is limited to maximally approx. 60 % of the pressure prevailing in the reactor containment (2).

18. The method of claim 17, wherein the vacuum is spontaneously generated through fast opening valves and a displaced volume from a vacuum/recirculation container.

19. The method of claim 17 or 18, wherein, after a sample has been taken, a pressure change with backflushing of the analyzing devices and the sampling line is carried out.

20. The method of any of claims 17 to 19, wherein the sample volume per individual analysis is limited to < 1 liter and/or to an activity content of < 10¹⁰ Bq.

21. The method of any of claims 17 to 20, wherein the flowing of the sample into the sampling line (8) is throttled.

22. The method of any of claims 17 to 21, wherein, in case of partial vapor pressures of several bars at the sampling spots, a falling below the dew point in the measured gas is avoided through a pressure decrease in the analyzing unit to preferably 1 bar.

23. The method of any of claims 17 to 22, wherein an additional measured value for the hydrogen concentration in the reactor containment (2) is determined in accordance with the heat-tone method.

## Revendications

1. Installation nucléaire, ayant une enceinte de sécurité (2) et ayant un système de prélèvement (1, 1') conçu pour extraire un échantillon de l'atmosphère à l'intérieur de l'enceinte de sécurité (2), le système de prélèvement (1, 1') comprenant une conduite de prélèvement (8) reliée avec un système de dépression (12) et avec un système d'analyse (14) et débouchant dans l'enceinte de sécurité (2), **caractérisé en ce**
**qu'**un dispositif d'étranglement (30) est relié en amont de la conduite de prélèvement (8) du côté du gaz lorsque la conduite de prélèvement (8) est reliée avec l'atmosphère à l'intérieur de l'enceinte de sécurité (2).

2. Installation nucléaire selon la revendication 1, dans laquelle la conduite de prélèvement (8) est conçue comme une petite conduite avec un diamètre nominal de jusqu'à 15 mm, préférablement comme une conduite capillaire avec un diamètre nominal d'environ 1 à 5 mm.

3. Installation nucléaire selon la revendication 1 ou 2, dans laquelle le dispositif d'étranglement (30) a une section libre de courant de 0,05 à 2 mm, préférablement d'environ 0,5 mm.

4. Installation nucléaire selon l'une quelconque des revendications 1 à 3, dans laquelle une unité de filtrage (32) est affectée au dispositif d'étranglement (30).

5. Installation nucléaire selon l'une quelconque des revendications 1 à 4, dans laquelle le système d'analyse (14) est prévu d'un boîtier extérieur (16) chauffable.

6. Installation nucléaire selon l'une quelconque des revendications 1 à 5, dans laquelle une isolation extérieure dans la zone du système d'analyse (14) limite les pertes de chaleur à moins de 100 W/m², préférablement à moins de 50 W/m².

7. Installation nucléaire selon l'une quelconque des revendications 1 à 6, dans laquelle une alimentation en courant électrique est prévue, qui comprend un réseau de batterie et/ou des groupes diesel-électrogène de secours séparés, comme protection contre des pannes d'électricité.

8. Installation nucléaire selon l'une quelconque des revendications 1 à 7, dans laquelle une unité de commande numérique, librement programmable, est affectée au système de prélèvement (1, 1'), à l'effet de commande et de réglage.

9. Installation nucléaire selon l'une quelconque des revendications 1 à 8, dans laquelle la conduite de prélèvement (8) est posée dans un tube protecteur.

10. Installation nucléaire selon l'une quelconque des revendications 1 à 9, dans laquelle le système de dépression (12) est placé à l'extérieur de l'enceinte de sécurité (2).

11. Installation nucléaire selon l'une quelconque des revendications 1 à 10, dans laquelle un récipient d'isolation d'échantillon (54) est connecté dans la conduite de prélèvement (8) avant son entrée dans le système d'analyse (14).

12. Installation nucléaire selon la revendication 11, dans laquelle le volume intérieur du récipient d'isolation d'échantillon (54) est de deux à cinq fois inférieur à un volume tampon connecté en amont.

13. Installation nucléaire selon l'une quelconque des revendications 1 à 12, dans laquelle la conduite de prélèvement (8) est chauffable dans une zone à l'extérieur de l'enceinte de sécurité (2).

14. Installation nucléaire selon l'une quelconque des revendications 1 à 13, dans laquelle le système d'analyse (14) comprend un nombre de capteurs capacitifs au polymère et/ou de détecteurs de conduction thermique pour analyser le gaz pour les composantes hydrogène et/ou contenu en vapeur et/ou monoxyde de carbone.

15. Installation nucléaire selon l'une quelconque des revendications 1 à 14, dans laquelle une conduite de recyclage (40) débouchant dans l'enceinte de sécurité (2) est reliée avec la conduite de prélèvement (8).

16. Installation nucléaire selon l'une quelconque des revendications 1 à 15, ayant un nombre de capteurs d'hydrogène (94) conçus selon le principe de la chaleur de réaction, qui sont placés à l'intérieur de l'enceinte de sécurité (2) et qui sont reliés du côté de données avec une unité d'évaluation externe (96) et au moyen de cette dernière, avec une unité d'évaluation électronique (26) commune pour le système de prélèvement (1, 1').

17. Procédé pour extraire un échantillon de l'atmosphère à l'intérieur d'une enceinte de sécurité (2) d'une installation nucléaire, dans lequel une pression plus basse que la pression régnant dans l'enceinte de sécurité (2) est générée dans une conduite de prélèvement (8),
**caractérisé en ce que**,
après que l'échantillon s'est écoulé dans la conduite de prélèvement (8), la pression dans la conduite de prélèvement (8) est limitée à environ 60 % au maximum, de la pression régnant dans l'enceinte de sécurité (2).

18. Procédé selon la revendication 17, dans lequel la dépression est générée spontanément à l'aide de vannes à ouverture rapide et d'un volume aspiré d'un récipient de dépression/de recyclage.

19. Procédé selon la revendication 17 ou 18, dans lequel, après le prélèvement d'un échantillon, il s'effectue un changement de pression avec rétrolavage des dispositifs d'analyse et de la conduite de prélèvement.

20. Procédé selon l'une quelconque des revendications 17 à 19, dans lequel le volume d'échantillon de chaque analyse individuelle est limité à < 1 litre et/ou à un contenu d'activité de < 10¹⁰ Bq.

21. Procédé selon l'une quelconque des revendications 17 à 20, dans lequel l'écoulement de l'échantillon dans la conduite de prélèvement (8) est étranglé.

22. Procédé selon l'une quelconque des revendications 17 à 21, dans lequel, en cas de pressions de vapeur partielles de plusieurs bars aux points de prélèvement, un sous-dépassement du point de condensation dans le gaz mesuré est évité par une réduction de pression dans l'unité d'analyse à préférablement 1 bar.

23. Procédé selon l'une quelconque des revendications 17 à 22, dans lequel une valeur mesurée additionnelle de la concentration en hydrogène dans l'enceinte de sécurité (2) est déterminée selon le procédé de la chaleur de réaction.
